**Europäisches Patentamt**

**European Patent Office**

**Office européen des brevets**

(19)

(11) Publication number: **0 193 831**
A1

## (12) EUROPEAN PATENT APPLICATION

(21) Application number: **86102380.2**

(22) Date of filing: **24.02.86**

(51) Int. Cl.⁴: **A 61 N 5/02,** H 01 Q 13/00, H 01 P 5/08, A 61 H 39/06

(30) Priority: **26.02.85 GB 8504935**

(43) Date of publication of application: **10.09.86**
**Bulletin 86/37**

(84) Designated Contracting States: **AT BE CH DE FR IT LI LU NL SE**

(71) Applicant: **NORTH CHINA RESEARCH INSTITUTE OF ELECTRO-OPTICS, P.O.Box 8511, Beijing East Suburbs (CN)**

(72) Inventor: **Chen, Zufan, P.O. Box 8511-40, Beijing (CN)**

(74) Representative: **Michelotti, Giuliano et al, c/o SAIC BREVETTI S.r.l. Viale Bianca Maria 15, I-20122 Milano (IT)**

(54) **Microwave therapeutic apparatus.**

(57) A wide bandwidth multi-frequency direct contact type microwave physiotherapeutic apparatus for replacing acupuncture therapy in medical and hygienical practice comprises a microwave generator with a multi-frequency output varying within a range of 100–3,000 MHz, and a non-invasive applicator (20) made up of an inner conductor (22) and an outer conductor (21) that both keep direct contact with skin during treatment while the exponentially flared outer conductor (21) serves as a screening structure to ensure a good match between the applicator and the body and to avoid microwave leakage to the environment.

The present invention relates to an apparatus for localised treating of the human or animal body using multi)frequency composite microwave radiation, which can substitute the traditional acupuncture therapy in medicine and hygiene.

There are various kind of microwave apparatus in prior art having different structures and characters, working by different processes and methods yet bearing the common feature of using the heating effect in living tissue induced by microwave radiation to realize medical purposes, therefore most of them being used, mainly in thermal treatment hyperthermia and diathermy, for example of cancer.

In recent years, Chinese researchers have put forward some new ideas on the physical effects of microwave raditioan on the human body. They hold that besides the thermal and nonthermal effects, there is a third effect produced during the microwave treatment of the human body, the regulation effect on the "jingluo" system or the channels and collaterals system of the human body. These ideas are based on the results of research work on the "jingluo" theory of traditional Chinese medicine, the mechanism of Qi Gong and Qi Gong therapy.

According to the theory of traditional Chinese medicine, there is a jingluo system coexisting inside the human body with the other physiological systems with anatomic structure like the circulating system, the nervous system etc. but which has not been found anatomically. A kind of "Qi" or vital energy moving along the jingluo decides the conditions of human health. The jingluo distribute themselves throughout the human body in their own way, forming along themselves specific positions for acupuncture treatment, called the "acupoints" or "points". It is according to this theory that the acupuncture therapy selects different points to cure a wide variety of diseases and illnesses in medical and hygienic practice. Another practice related to the same theory is Qi Gong, a kind of deep breathing exercises. Having been trained and taken up the exercise for a long time, a Qi Gong master can reach such a state that when he concentrates himself in the exercise, he can use his mind to control the

movement of the vital energy along the jingluo through his body, thus to keept himself in good health. Some masters even can send out the vital energy through a certain point, this is called "Fa Gong" or energy emitting. Such a master can cure a patient by emitting the "energy" through a point, for example on his hand, to a selected point on the patient's body. This clinical practice of energy emitting therapy or Qi Gong therapy, has long been practised and widely acknowledged in China. Nevertheless a problem ramains that there are only a few Qi Gong masters who can reach such a state, therefore the therapy cannot be applied widely.

Some researches believe that there is some kind of relation between the jingluo system and the electromagnetic filed of the human body. By studying the energy emitting phenomena with the help of modern equipments, researchers have detected at the energy emitting point existance of wide band infrared radiation and low frequency subsonic waves, enhancement of magnetic filed density and concentration of static electric filed. The relevant testing results were published in a report on page 16 of "The Infrared Observer", No. 2/80 with the inventor of the present invention doing the work described.

In the light of the aforesaid results, some researchers believe that the health conditions of human beings may be improved by regulating the electromagnetic field of the human body through various ways of stimulating points, such as acupuncture, energy emitting etc.. Since microwave radiation can go deep into the body and produce many kinds of physical effects, it is belived that the stimulation of the points by microwave radiation can achieve effects similar to that of acupuncture, moxibustion and Qi Gong therapy.

As the biological electromagnetic field has a feature of continuous distribution and fluctuation, multifrequency composite microwave radiation should be used for point treatment with concentrated microwave energy irradiating local surface areas of the human body, so that good results of physical treatment similar to that of acupuncture and Qi Gong therapy, yet not obtainable by prior microwave treatment, can be obtained.

0193831

Based on the aforesaid inventive idea, the same inventor first invented; before the present invention, an apparatus for microwave acupuncture treatment, published by the inventor in an article on page 89 of January issue of ACTA ELECTRONICA SINICA, 1982. The apparatus consists of an acupuncture antenna and a microwave generator. The said antenna includes an outer conductor of spiral shaped wire and an inner conductor which is an acupuncture needle. The needle is stuck into a selected point during treatment and the spiral wire is kept in contact with the skin around the point. The said inner and outer conductors are coupled with the human body directly while microwave radiation is emitted quantitatively and directionally to the point. Statistical results of clinical practice showed that this apparatus is more effective than the traditional acupuncture therapy. It has been well accepted and won commercial success on the market and a gold medal was awarded at the fortieth Plovdiv International Technology Exhibition held in Bulgaria in 1984. Yet some patients do not like to receive the treatment because of the pain created when the needle is stuck in. This remains a difficult problem in traditional acupuncture therapy. Moreover, the fixing of the antenna during treatment makes the patient uneasy. There exists considerable leakage during operation by the spiral outer conductor of the microwave antenna. The difficulty encountered in matching the antenna's output impedance with that of the human body further reduces the efficiency of the apparatus. Besides, the output frequency range of the said microwave generator is limited leading to less frequency components in the composite wave which is undesirable for achieving a multi-frequency composite wave output, a requirement put forward by the original design. The antenna with such a design limits the use of the apparatus to acupuncture treatment only.

The present invention is an improvement to the aforesaid apparatus for microwave acupuncture treatment. To solve the problem presented in prior art, a new microwave applicator is designed, comprising an inner conductor of metal bar and a screening outer conductor formed around the inner

conductor with one end open. The two conductors are fixed on the same base structure, insulated from each other, and connected respectively trough the base with the inner and outer conductors of a coaxial cable.The top of the metal bar and the open end of the screening structure together from the therapeutic head of the microwave applicator and the said head contacts directly with the skin during treatment with the outer conductor forming a screening structure. The present invention improves the original microwave generator in that its output frequency bandwidth is extended to 100-3,000 MHz and the number of components comprising its multiwave output is increased. In order to realize the match of impedence between the applicator and the human body for all frequency components, the said outer conductor may be a special geometric shape, which increases the output efficiency, reduces the microwave leakage and avoids substantially the microwave interference to the environment. This non-invasive applicator avoids the penetration involved in acupuncture and diminishes the pain and the danger of cross-infection of the patient. Furthermore it limits the area and dosage of irradiation, thus causing no by effect to the patient. Since the applicator can be easy fixed onto the right place on a body, it allows the patient the possibility of moving slightly during treatment. By changing the dimensions of the inner and outer conductors, the applicator can be used in hyperthermia and diathermy. The inner conductor can also be replaced by a needle in treatment, thus maintaining all the merits of the original microwave acupuncture apparatus. The present invention can also be used for treatment of animals according to the same principle.

Embodiments of the present inveniton will now be described with reference to the accompanying drawings, in which:

Fig. 1 illustrates schematically the apparatus for microwave acupuncture treatment in prior art;

Fig. 2 illustrates schematically the inner conductor of the antenna of the microwave acupuncture apparatus shown in fig. 1;

Fig. 3 illustrates the output frequency distribution of the apparatus shown in fig. 1;

Fig. 4 illustrates the output frequency distribution of the apparatus shown in fig. 9;

Fig. 5 illustrates schematically the microwave applicator according to this invention;

Fig. 6 illustrates schematically the applicator used together with a needle;

Fig. 7 illustrates the distribution of the electromagnetic field of the applicator when it is working;

Fig. 8 illustrates another embodiment of the applicator; and

Fig. 9 illustrates an embodiment of the invention including a microwave generator and an applicator connected together.

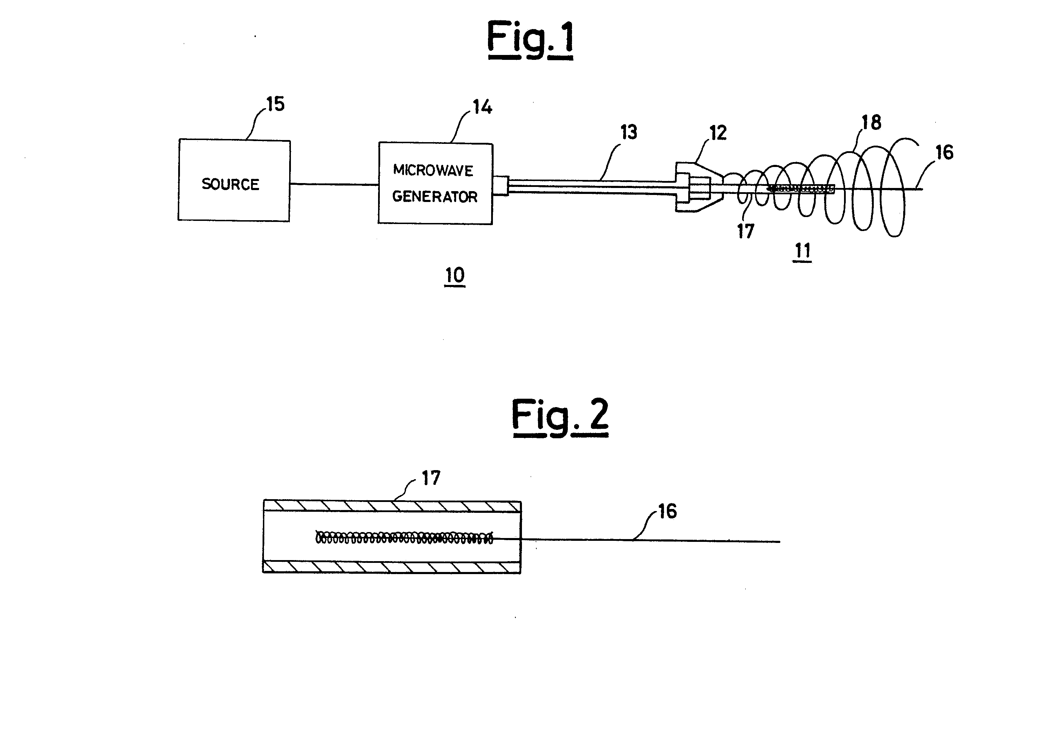

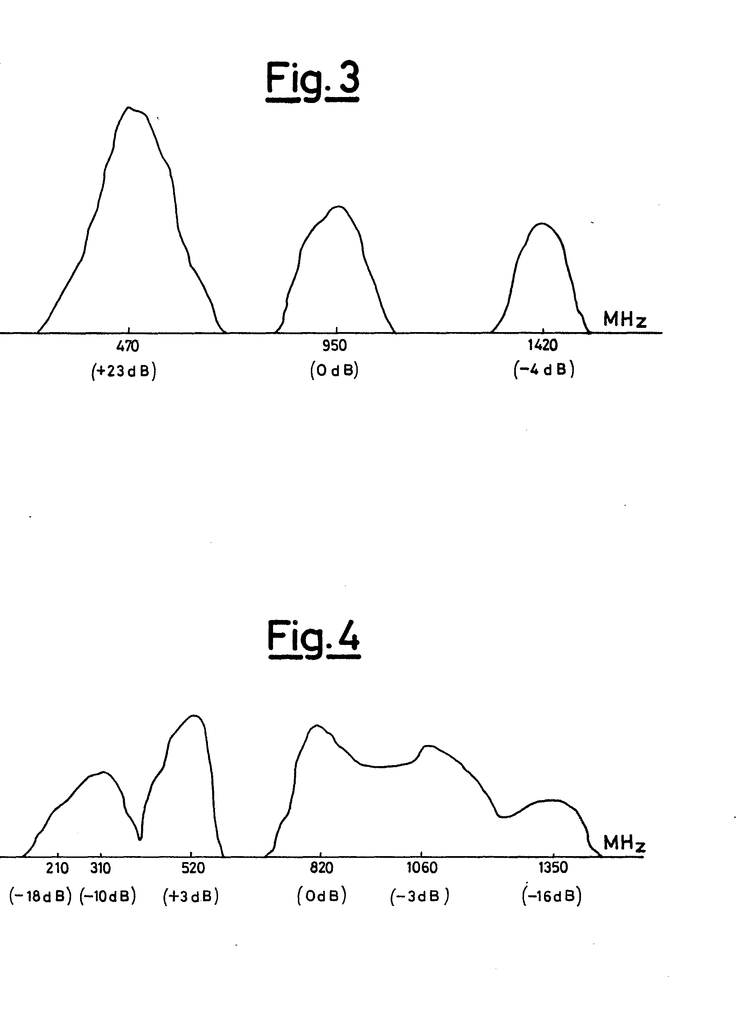

Fig. 1 illustrates schematically the apparatus for microwave acupuncture treatment in prior art, wherein the said apparatus 10 comprises an acupuncture antenna 11, a coaxial cable 13, a microwve generator 14 and a power source 15. The acupuncture antenna has a base 12, an outer conductor of spiral shaped wire 18, an inner conductor of a tube 17 and a needle 16. The inner conductor 17 and the outer conductor 18 are insulated from each other and the handle of the needle 16 is situated inside the tube of the inner conductor, as shown in fig. 1. When giving a physiotherapeutic treatment, first stick the needle into the point, then slip the handle of the needle into the inner conductor 17 and form a capacitive coupling between them while keeping the outer conductor in contact with the skin around the point. The power source 15 can be any kind of DC source and the output power of the antenna is controlled by adjusting the output voltage of the source. The output frequency distribution of the microwave generator 14 is shown in fig. 3.

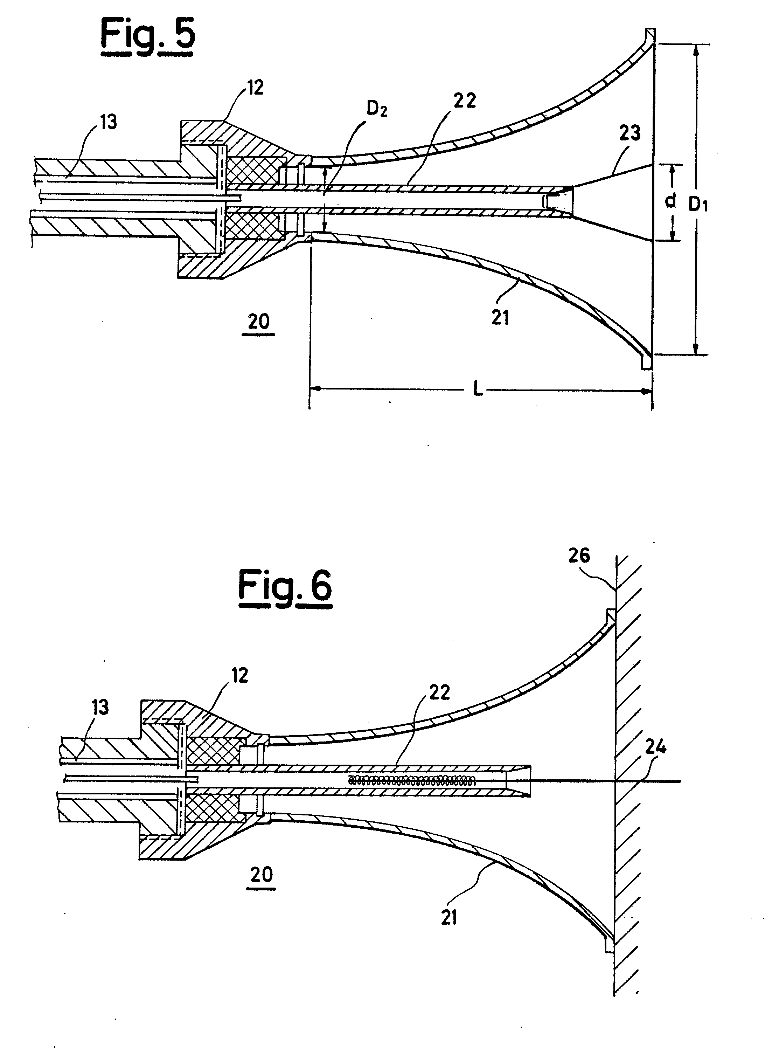

Fig. 5 shows the microwave applicator of this invention. The applicator 20 comprises an coter conductor 21, an inner conductor 22 and a base 12. The base 12 and the coaxial cable 13 connected with it are similar to that shown in fig. 1. One end of the inner conductor 22 is fixed on the base 12 and is connected to the inner conductor of the cable 13 while the other serves as a physiotherapeutic head 23, which is made of metal and can be separated from the inner conductor 22. The outer conductor 21 is also fixed on the base 12 and is connected to the outer conductor of the cable 13. The inner and outer conductors 21 and 22 are insulated from each other. The outer conductor 21 expands exponentially from the base end to the open end to provided good impedance match between the applicator and human body at all freaquencies of the composite wave. The cross section of the outer conductor can be any form of a closed curve, but preferably that of a circle. When the cross section of both the inner and outer conductors are circular, as shown in fig. 5 with $D_1$ representing diameter of the open end of the outer conductor, $D_2$ representing diameter of the base end of the outer conductor, L representing lenght of the outer conductor, d representing diameter of the physiotherapeutic head of the inner conductor. The shape of the outer conductor can then be determined by the following equation:

$$y = ae^{bx} \quad \text{wherein } a = D_2/2 \quad b = (1/L)\ln(D_1/D_2).$$

When the shape of the outer conductor has been determined, the output impedance of the applicator can be calculated by the following equation:

$$Z_o = (60/\sqrt{\varepsilon})\ln(D_1/d) \quad \text{wherein } \varepsilon \text{ represents dielectric constant of the}$$

skin. In practice $D_2$ can be determined from the dimensios of the coaxial cable and the conventional connector selected, L can be of any value chosen mainly for the purpose of convenience, while $D_1$ and d are determined by the use and the position of treatment on the body and can be changed at any time. The ranger of their dimensions are as follows:

$$d: 0.1\text{--}45.0 \text{ mm} \qquad D_1: 10\text{--}200 \text{ mm}.$$

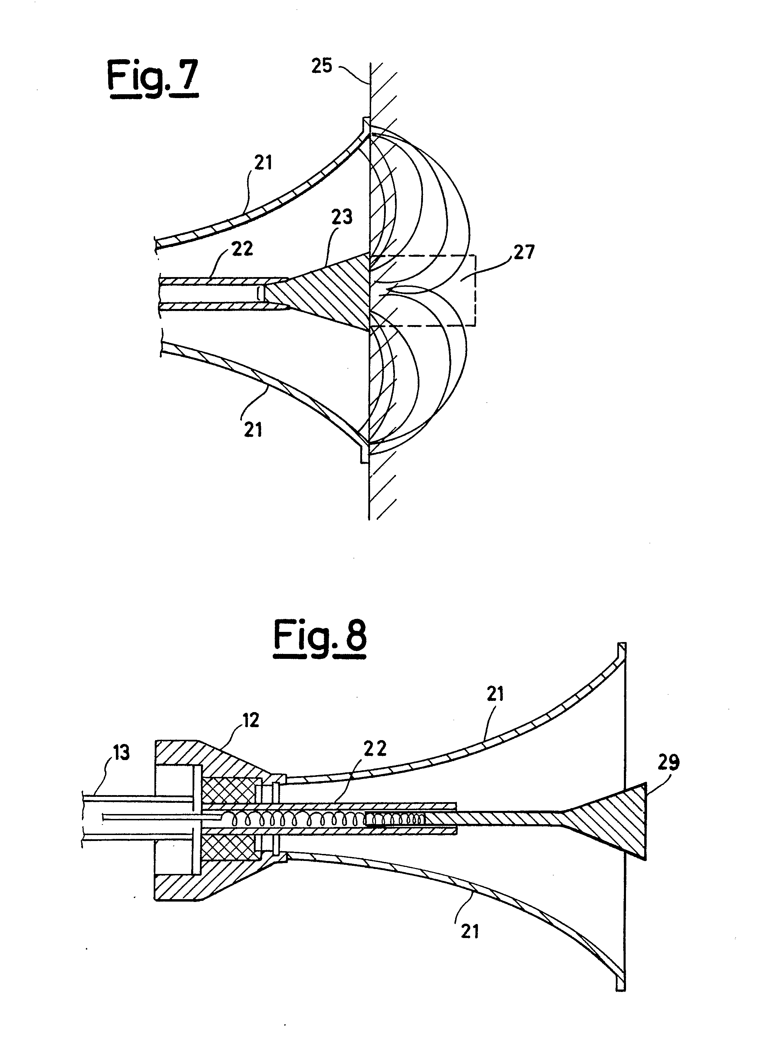

When a physiotherapeutic head 23 with a d of a small value is used to irradiate microwave energy to a point, a localized area of high density electromagnetic field is formed, as shown in fig. 7 where the physiotherapeutic head 23 contacts with the skin, and the microwave energy irradiated on this area will go deep into the body. In addition, since the output from the head 23 is a composite wave of multi-frequency the depth of penetration of each frequency component differs from one ano-

ther. As a result, a vertical distribution of stimulation at the point is formed, which enables this apparatus to obtain, without the use of a needle, an effect very similar to that of acupuncture. The apparatus can be used to replace the conventional acupuncture therapy with a similar curative effect by providing the said inner conductor with a head of small d. When an applicator with large $D_1$ and d is used, an electromagnetic field of a relatively even density forms, enabling the apparatus to be used in hyperthermia an diathermy. For patients preferring the acupuncture treatment, the apparatus can be used in combination with an acupuncture needle by removing the head 23, as shown in fig. 6. With the use of the present apparatus, the patient can receiving simultaneously the effects of acupuncture, moxibustion, hyperthermia and diathermy as a result of the combined heating effect and skin effect, as shown in fig. 6. Preliminary statistic results of clinical practice have proved that this combined physiotherapeutic treatment is more effective than the conventional acupuncture treatment. Moreover owing to the screening effect of its outer conductor and the good impedance match between the applicator and the human body, the present apparaus as compared with the microwave acupuncture antenna of spiral wire outer conductor in prior art, has a smaller output loss and almost no microwave interference to the environment. In addition, the capacitive coupling between the inner conductor and the needle provides at the point of the needle a relatively strong discharge which produces a localised high temperature area. Such a feature enables the present apparatus to be used for other medical purposes, for example, the thermal treatment of cancer.

Fig. 8 shows another embodiment of this invention, wherein the physiotherapeutic head 29 has an elastic connection with the inner conductor 22. The advantage of this design is that during treatment the inner conductor is kept in close contact with the point to provide a good match between the applicator and the body. This design is particularly useful for such special point as those on hands, and for very thin patients. One or more slots can be opened vertically on the outer conductor for letting out the sweat moisture.

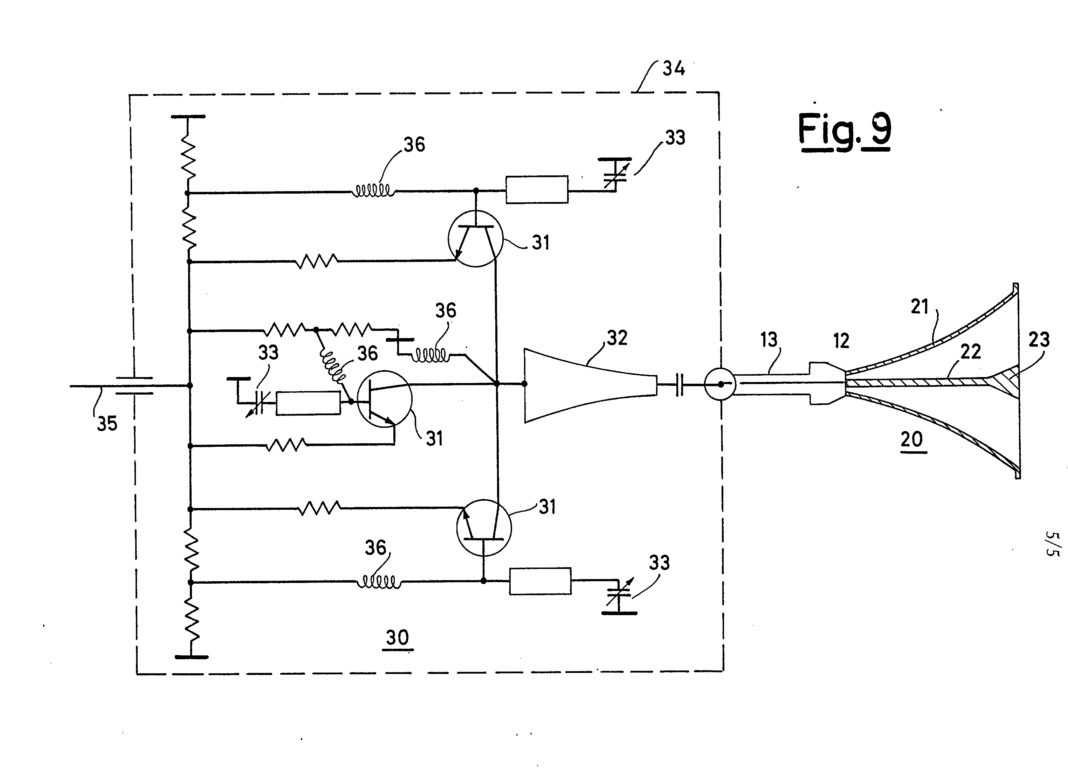

Fig. 9 shows the circuit structure of the invention, wherein the applicator 20 can be any one shown in Fig. 5,6 and 8. The microwave generator 30 comprises three transistor oscillators connected in parallel and an impedance transformer 32. The three transistors 31 can be of the same or different type. The impedance transformer 32 is a microstrip impedance transformer, which is connected between the oscillators and the coaxial cable 13. The width of the transformer reduces exponentially from the oscillator end to the cable end. This ensures a good match at all frequencies of the composite wave. Oscillating frequency can be changed by adjusting the parameters of the inductor 36 and/or the variable capacitor 33 within the range of 100-3,000 MHz. The oscillating frequencies of the three oscillators differ from one another. The simultaneously parallel output of the three oscillators through the impedance transformer 32 makes possible the multi-frequency output of the apparatus. The output frequency profile of this apparatus is shown in fig. 4 which is only an example and the profile can be modified by regulating circuits parameters. According to fig. 9, the number of frequency components can be increased by increasing the number of parallel oscillators within the cost-effective range. The present invention can be fulfilled by replacing the transistor oscillators with other component parts, for example integrated circuits to induce the microwave generator. The microwave generator 30 in fig. 9 is connected to the DC source through lead 35 and the earth in the circuit. The DC source can be a battery or a stabilized DC source and the adjustment of the DC voltage by conventional methods can change the output power of the apparatus within a range of 0.1-3 watt. The dashed line 34 in fig. 9 represents an earthed metal layer which provides goods screening for the generator, avoides microwave interference with the environment and reduces the leval of microwave radiation received by the operators, at the same time, it serves as a heat sink for the microwave generator as well.

0193831

CLAIMS

1. A microwave therapeutic apparatus comprising: a microwave generator, (30) a microwave applicator (20) connected to the generator (30) for emitting microwaves to a localised area on the human and animal body, characterized in that the applicator (20) comprises an inner conductor (22) and an outer conductor (21) in a design that both of the conductors keep direct contact with skin (26) during treatment, with the outer conductor (21) serving as a screening structure.

2. An apparatus according to claim 1, characterized in that (30) the generator comprises:

- a plurality of parallely-connected oscillators of different frequencies with a common output line connected to the applicator (20) for emitting multiwaves within a frequency range of 100 to 3,000 MHz.

3. An apparatus according to claim 2, characterized in that (30) the generator further comprises:

- a microstrip impedance transformer (32) connected between the oscillators and the applicator, (20) with its width reducing exponentially from the oscillators' end to the applicator's end.

4. An apparatus according to claim 1, characterized in that the applicator (20) comprises an inner conductor (22) of a metal bar connected to the output line of the generator at one end with the other end serving as a physiotherapeutic head, (23) and an outer conductor (21) of a screening structure connected to the earth of the generator at one end and opened at the other end with the inner conductor (22) being held inside its structure.

5. An apparatus according to claim 4, characterized in that the inner conductor (22) has a physiotherapeutic head 29) separable from and fixable to the inner conductor (22).

6. An apparatus according to claim 5, characterized in that the physiotherapeutic head (29) is connected elastically to the inner conductor (22) and can make radial elastic movement relative to the inner conductor (22).

7. An apparatus according to claim 5 or claim 6, wherein the inner conductor (22) is of a hollow structure than can hold the handle of an acupuncture needle (24) when the physiotherapeutic head (23) is removed and can form a capacitive coupling with the handle during treatment while keeping the outer conductor (21) in contact with the skin (26).

8. An apparatus according to claim 4, claim 5, claim 6 or claim 7, characterized in that the outer conductor (21) expands exponentially from the end connected to the generator to the open end and has a cross section in the form of any substantially closed curve.

9. An apparatus according to claim 8, characterized in that the cross section of the outer conductor (21) is circular and the exponential curve conforms to the following equation:

$y = a\,e^{bx}$ wherein $a = D_2/2$, $b = (1/L)\ln(D_1/D_2)$,

$D_1$ representing the diameter of the open end of the outer conductor, $D_2$, representing the diameter of the other end of the outer conductor, L represents the length of the outer conductor.

10. An apparatus according to any one of claims 4 to 9, wherein both the inner conductor (22) and the outer conductor (21) have circular cross sections, and the diameter of the physiotherapeutic head (23), d, and the diameter of the open end, $D_1$, are within the following ranges:

d: 0.1 - 45.0 mm. $D_1$: 10-200 mm.

# Fig.1

SOURCE 15

MICROWAVE GENERATOR 14

13

12

18

16

17

11

10

# Fig.2

17

16

## Fig.3

MHz

470
(+23 dB)

950
(0 dB)

1420
(−4 dB)

## Fig.4

MHz

210
(−18 dB)

310
(−10 dB)

520
(+3 dB)

820
(0 dB)

1060
(−3 dB)

1350
(−16 dB)

Fig.5

Fig.6

## Fig.7

## Fig.8

Fig. 9

## DOCUMENTS CONSIDERED TO BE RELEVANT

EP 86102380.2

| Category | Citation of document with indication, where appropriate, of relevant passages | Relevant to claim | CLASSIFICATION OF THE APPLICATION (Int. Cl.4) |
|---|---|---|---|
| X | WO - A1 - 80/1461 (BSD) | 1,2,4-6,8,10 | A 61 N 5/02 |
| A | * Abstract; page 16, lines 1-8; page 17, lines 16-29; fig. 2, 4 * | 7 | H 01 Q 13/00 |
| | | | H 01 P 5/08 |
| | -- | | A 61 H 39/06 |
| X | FR - E - 60 161 (SOCIETE PARISIENNE D'EXPLOITATION) | 1,4 | |
| | * Fig. 1,2 * | | |
| | -- | | |
| X | US - A - 4 041 499 (LIU) | 1 | |
| | * Abstract; column 2, lines 32-45; fig. 1,2 * | | |
| | -- | | |
| Y | DE - A1 - 3 009 734 (LINDENMEIER) | 1 | |
| A | * Pages 1,2, claim 1; fig. 1 * | 4 | TECHNICAL FIELDS SEARCHED (Int. Cl.4) |
| | -- | | |
| Y | DE - A1 - 2 736 380 (BOSCH) | 1 | A 61 N |
| A | * Page 8, lines 10-17; fig. * | 7 | H 01 Q |
| | -- | | H 01 P |
| X | EP - A1 - 0 044 538 (OLYMPUS) | 1,4 | A 61 H |
| | * Abstract; page 5, line 15 - page 6, line 10; fig. 4 * | | |
| | -- | | |
| A | US - A - 3 623 118 (RAYTHEON) | 1 | |
| | * Fig. 1 * | | |
| | -- | | |
| A | GB - A - 1 418 675 (RCA) | 1-3 | |
| | * Page 3; claim 1 * | | |
| | -- | | |

The present search report has been drawn up for all claims

| Place of search | Date of completion of the search | Examiner |
|---|---|---|
| VIENNA | 27-05-1986 | NEGWER |

## EUROPEAN SEARCH REPORT

**European Patent Office**

### DOCUMENTS CONSIDERED TO BE RELEVANT

EP 86102380.2

| Category | Citation of document with indication, where appropriate, of relevant passages | Relevant to claim | CLASSIFICATION OF THE APPLICATION (Int. Cl.4) |
|---|---|---|---|
| A | GB - A - 2 089 135 (MARCONI) <br> * Abstract; fig. 1 * <br> ---- | 1-3 | |

TECHNICAL FIELDS SEARCHED (Int. Cl.4)

The present search report has been drawn up for all claims

| Place of search | Date of completion of the search | Examiner |
|---|---|---|
| VIENNA | 27-05-1986 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82